# EUROPÄISCHE PATENTSCHRIFT

(11) **EP 0 714 632 B1**
(45) Veröffentlichungstag und Bekanntmachung des Hinweises auf die Patenterteilung: **07.05.1997**
(21) Anmeldenummer: 95116006.8
(22) Anmeldetag: 11.10.1995
(51) Int. Cl.: A61B 6/14, A61B 1/247

(54) **Gerät zur Bilderfassung im Oralbereich, insbesondere zur zahnmedizinischen Diagnose**
Device for the acquisition of image data from the oral region to be used, in particular for dental diagnosis
Dispositif de captation d'images de la région buccale destiné, en particulier au diagnostic dentaire

(30) Priorität: 30.11.1994 DE 4442611
(43) Veröffentlichungstag der Anmeldung: 05.06.1996
(73) Patentinhaber: Pfeiffer, Manfred, Dr., London W9 1EL (GB)
(72) Erfinder: Pfeiffer, Manfred, Dr., London W9 1EL (GB)
(74) Vertreter: Stenger, Watzke & Ring Patentanwälte

(56) Entgegenhaltungen:
- EP-A- 0 544 974
- DE-A- 3 932 845
- DE-C- 4 218 020
- US-A- 4 160 997

## Beschreibung

Die Erfindung betrifft ein Gerät zur Bilderfassung im Oralbereich, insbesondere zur zahnmedizinischen Diagnose, mit einem Gehäuse sowie einem in dem Gehäuse angeordneten, flachen Bildsensor, dessen Bildsignale über eine Signalleitung an eine getrennt angeordnete Bildverarbeitungs- und Speichereinheit übertragbar sind, wobei sich der Bildsensor aus matrixförmig auf einem Substrat angeordneten, optoelektronischen Halbleiterelementen, sowie aus einem Ausleseregister zusammensetzt, welches die in den Halbleiterelementen in Abhängigkeit von der jeweils empfangenen Strahlungsintensität gespeicherten Ladungen erfaßt. Ein solches Gerät ist aus US-A-4 160 997 bekannt.

Derartige Geräte werden unter dem Fachbegriff "Intraoralsensor" zusammengefaßt. Sie werden vom Zahnarzt verwendet, um Röntgenaufnahmen von Zähnen und kleineren Zahngruppen anzufertigen. Hierzu wird das Gehäuse des Sensors in den Mund des Patienten eingeführt und hinter dem zu durchleuchtenden Zahn oder der Zahngruppe positioniert, wobei der flach in dem Gehäuse ausgebildete Bildsensor mit seiner strahlungsempfindlichen Schicht zu dem Zahn bzw. zu der Zahngruppe hinweist. Anschließend erfolgt von außerhalb die Beaufschlagung mit Röntgenstrahlen in geringer Dosis, wobei die auf den Bildsensor auftreffenden optischen Impulse über eine aus dem Gehäuse herausgeführten Signalleitung zu einer Bildverarbeitungs- und Speichereinheit gelangen. Dies ist in der Regel ein Personal-Computer, der über eine Bildverarbeitungs-Software verfügt. Auf diese Weise können Röntgenbilder an Ort und Stelle sowie verzögerungsfrei aufgenommen und auf dem Bildschirm des Computers sichtbar gemacht werden. Der behandelnde Zahnarzt kann dann selbst entscheiden, welche ergänzenden Bilder eventuell noch erforderlich sind.

Nachteilig bei den bekannten Geräten ist, daß diese über relativ eckige bzw. kantige Gehäuse verfügen. Hierdurch kann es beim Patienten zu Druckstellen und damit Schmerzen im Bereich der Mundhöhle oder des Zahnfleisches kommen. Die Gestaltung des Gehäuses läßt sich auch nicht ohne weiteres den Gegebenheiten in der Mundhöhle anpassen, da sich die Gestaltung der Gehäuseoberfläche nach der Form des darin enthaltenen Bildsensors zu richten hat. Der Bildsensor wiederum unterliegt engen gestalterischen Grenzen, da an zumindest einer Kante der Sensorfläche das Ausleseregister angeordnet ist.

Der Erfindung liegt die **Aufgabe** zugrunde, ein Gerät zur Bilderfassung im Oralbereich und insbesondere zur zahnmedizinischen Diagnose zu schaffen, welches dem Patienten während der Anwendung ein besseres Tragegefühl vermittelt.

Zur **Lösung** dieser Aufgabe wird bei einem Gerät zur Bilderfassung der eingangs genannten Art vorgeschlagen, daß das Ausleseregister innerhalb der Sensorfläche des Bildsensors angeordnet ist, so daß sich Halbleiterelemente zu beiden Seiten der Längsachse des Ausleseregisters befinden, daß die Sensorfläche als Rechteck mit gerundeten oder schräg geschnittenen Ecken gestaltet ist, und daß das Gehäuse an den den gerundeten oder schräg geschnittenen Ecken entsprechenden Stellen gerundet geformt ist.

Das erfindungsgemäße Gerät verwendet einen Bildsensor, bei dem sich das Ausleseregister innerhalb der Sensorfläche befindet, und nicht an deren Rand. Dadurch ist es möglich, die Randbereiche der Sensorfläche relativ frei zu gestalten. Diese können mit gerundeten oder schräg geschnittenen Ecken versehen werden, wodurch sich dann die Möglichkeit ergibt, auch das Gehäuse mit entsprechenden Rundungen zu versehen. Ein solches Gehäuse erzeugt bei der Anwendung im Zahnbereich des Patienten weniger Druckstellen als die bekannten, wesentlich eckiger gestalteten Sensorgehäuse, so daß sich der Tragekomfort insgesamt verbessert.

Weitere Einzelheiten eines erfindungsgemäß ausgebildeten Gerätes zur Bilderfassung im Oralbereich werden nachfolgend anhand der zugehörigen Zeichnung erläutert. In der Zeichnung zeigen:
- Fig. 1: in einer Ansicht ein Gerät zur Bilderfassung im Oralbereich mit einer aus Übersichtsgründen geschnittenen Zuleitung;
- Fig. 2: einen Schnitt entlang der Linie II-II der Fig. 1;
- Fig. 3: einen in dem Gerät nach den Figuren 1 und 2 verwendeten Bildsensor und
- Fig. 4: einen sehr stark vergrößerten Ausschnitt aus dem Bildsensor gemäß Fig. 3.

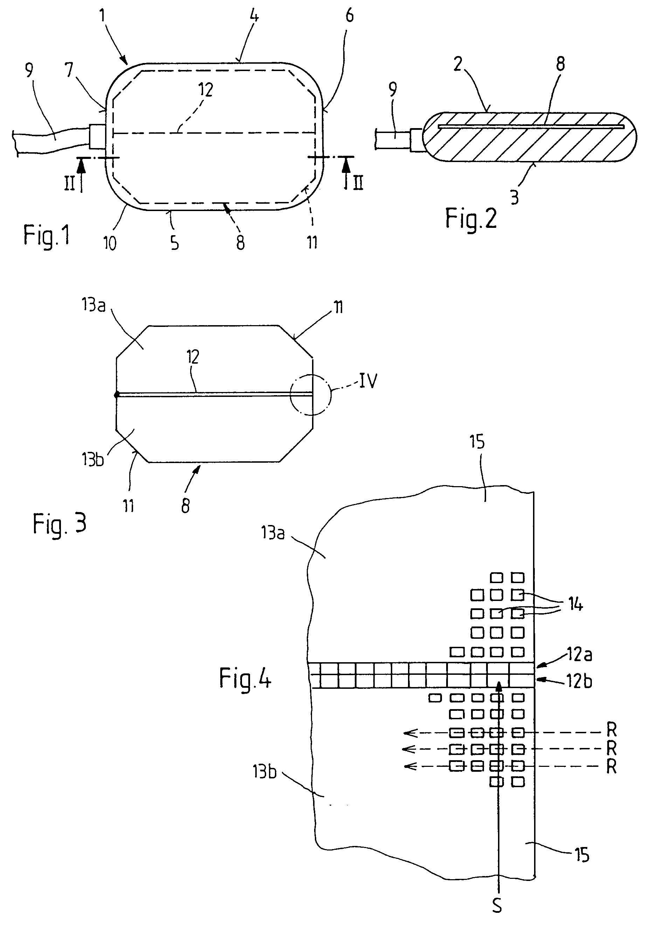

Das in den Figuren 1 und 2 dargestellte Gerät besteht aus einem im wesentlichen rechteckigen Gehäuse 1 mit Grundabmessungen von etwa 25 mm x 32 mm x 7,5 mm. Das Gehäuse 1 verfügt über zwei Hauptseiten 2,3, zwei längere Schmalseiten 4,5 und zwei kürzere Schmalseiten 6,7. Nahe der einen Hauptseite 2 ist in dem Gehäuse 1 ein auf Röntgenstrahlen sensibilisierter Bildsensor 8 angeordnet. Mittels einer Signalleitung in Form eines flexiblen Kabels 9 lassen sich die Signale des Bildsensors 8 einer auf der Zeichnung nicht dargestellten Bildverarbeitungs- und Speichereinheit zuführen. Ebenfalls auf der Zeichnung nicht dargestellt ist die in Richtung auf den Bildsensor 8 ausgerichtete Röntgenstrahlenquelle. Bei der Anwendung wird das Gerät mit dem nach vorne gerichteten Bildsensor 8 so in den Mund des Patienten eingesetzt, daß der zu durchleuchtende Zahnkieferbereich zwischen Röntgenquelle und Bildsensor 8 liegt. Die genaue Ausrichtung der Achsen der Röntgenstrahlenquelle einerseits und des Bildsensors 8 andererseits kann mittels bekannter Zentriereinrichtungen erfolgen.

Fig. 1 läßt erkennen, daß sämtliche Ecken des im wesentlichen rechteckigen Gehäuses 1 mit großzügigen Rundungen 10 versehen sind. Diese Rundungen 10 sind möglich, da der flach in dem Gehäuse 1 angeordnete Bildsensor 8 zwar im wesentlichen rechteckig ausgebildet ist, jedoch die Ecken 11 dieses Rechtecks im Bereich der Rundungen 10 des Gehäuses 1 schräg angeschnitten sind.

Der in Fig. 3 dargestellte Bildsensor 8 verfügt über eine durch ein Ausleseregister 12 zweigeteilte Sensorfläche. Beim Ausführungsbespiel bildet das Ausleseregister 12 die Längsachse der Sensorfläche, so daß sich beidseits des Ausleseregisters 12 jeweils sechseckige Teilflächen 13a, 13b ergeben. Der Bildsensor 8 als Ganzes ist achteckig ausgebildet.

Beim Ausführungsbeispiel befindet sich das Ausleseregister 12 symmetrisch in der Sensorfläche, teilt diese also in zwei spiegelbildliche Teilflächen 13a, 13b. Es ist aber ebenso möglich, das Ausleseregister 12 außermittig oder diagonal anzuordnen, so daß sich unterschiedlich große Teilflächen 13a,13b ergeben. Wesentlich ist allein, daß sich das Ausleseregister 12 nicht am Rand der Sensorfläche befindet, da dies die freie Gestaltbarkeit des Umrisses des Bildsensors 8 beeinträchtigen würde.

Die Funktion des Bildsensors 8 sowie die Bedeutung des Ausleseregisters 12 wird nachfolgend anhand der Fig. 4 erläutert. Einzelheiten finden sich z.B. in dem Fachbuch von Phillip E. Mattison: "Practical digital video with programming examples in C", erschienen im Verlag John Wiley & Sons, Inc.

Der im Ausführungsbeispiel dargestellte Bildsensor ist vom sogenannten "CCD"-Typ ("Charge Coupled Device"). Die Sensorfläche ist mit Bildpunkten ("Pixels") versehen, die in Reihen R und Spalten S angeordnet sind, so daß sich insgesamt die Struktur einer Matrix ergibt. Jeder Bildpunkt besteht aus einem diskreten Halbleiterelement 14, welches sich aus einem strahlungsempfindlichen Element, sowie einem Speicherelement zusammensetzt. Sämtliche Halbleiterelemente 14 sind auf einem gemeinsamen Substrat 15 aus Silikon angeordnet.

Parallel zu den Reihen R erstrecken sich zwei langgestreckte, parallel zueinander verlaufende Ausleseregister 12a, 12b. Das Ausleseregister 12a ist der Teilfläche 13a, und das Ausleseregister 12b ist der Teilfläche 13b des Bildsensors 8 zugewiesen.

Auf die einzelnen Halbleiterelemente 14 auftreffende Röntgenstrahlen führen dazu, daß in dem strahlungsempfindlichen Element des jeweiligen Halbleiterelementes 14 Ladungsteilchen frei werden, die in das zugehörige Speicherelement gelangen. Wird das betreffende Halbleiterelement 14 abgefragt, gelangen die so vorläufig gespeicherten Ladungsimpulse in das Ausleseregister 12a, 12b, um dann als Bildsignal weiterverarbeitet zu werden. Die einzelnen Halbleiterelemente 14 werden Reihe R für Reihe R durch das jeweilige Ausleseregister 12a,12b abgefragt, so daß die Leserichtung gleich ist der Ausrichtung der Spalten S.

### Bezugszeichenliste

- 1: Gehäuse
- 2: Hauptseite
- 3: Hauptseite
- 4: Schmalseite
- 5: Schmalseite
- 6: Schmalseite
- 7: Schmalseite
- 8: Bildsensor
- 9: Kabel
- 10: Rundung
- 11: Ecke, schräg angeschnitten
- 12: Ausleseregister
- 12a: Ausleseregister
- 12b: Ausleseregister
- 13a: Teilfläche
- 13b: Teilfläche
- 14: Halbleiterelement
- 15: Substrat
- R: Reihe mit Halbleiterelementen
- S: Spalte mit Halbleiterelementen

## Patentansprüche

1. Gerät zur Bilderfassung im Oralbereich, insbesondere zur zahnmedizinischen Diagnose, mit einem Gehäuse (1) sowie einem in dem Gehäuse (1) angeordneten, flachen Bildsensor (8), dessen Bildsignale über eine Signalleitung (9) an eine getrennt angeordnete Bildverarbeitungs- und Speichereinheit übertragbar sind, wobei sich der Bildsensor (8) aus matrixförmig auf einem Substrat (15) angeordneten, optoelektronischen Halbleiterelementen (14), sowie aus einem Ausleseregister (12) zusammensetzt, welches die in den Halbleiterelementen (14) in Abhängigkeit von der jeweils empfangenen Strahlungsintensität gespeicherten Ladungen erfaßt,
**dadurch gekennzeichnet,**
daß das Ausleseregister (12) innerhalb der Sensorfläche des Bildsensors angeordnet ist, so daß sich Halbleiterelemente (14) zu beiden Seiten der Längsachse des Ausleseregisters (12) befinden, daß die Sensorfläche als Rechteck mit gerundeten oder schräg geschnittenen Ecken (11) gestaltet ist, und daß das Gehäuse (1) an den den gerundeten oder schräg geschnittenen Ecken entsprechenden Stellen gerundet geformt ist.

## Claims

1. Device for acquiring images in the oral region, in particular for dental diagnosis, having a housing (1) and a flat image sensor (8) which is arranged in the housing (1) and whose image signals can be transmitted via a signal line (9) to a separately arranged image processing and storage unit, the image sensor (8) being composed of matrix-shaped optoelectronic semiconductor elements (14) arranged on a substrate (15), as well as of a read-out register (12) which detects the charges stored in the semiconductor elements (14) as a function of the respectively received intensity of radiation, characterized in that the read-out register (12) is arranged inside the sensor surface of the image sensor such that semiconductor elements (14) are located on both sides of the longitudinal axis of the read-out register (12), in that the sensor surface is shaped as a rectangle with rounded or obliquely cut corners (11), and in that the housing (1) is formed in a rounded fashion at the points corresponding to the rounded or obliquely cut corners.

## Revendications

1. Dispositif pour capter des images dans la région buccale, en particulier en vue d'un diagnostic dentaire, avec un étui (1) et un capteur d'images (8) plat, qui est disposé dans l'étui (1) et dont les signaux d'image peuvent être transmis par l'intermédiaire d'une ligne de transmission de signaux (9) à une unité séparée de traitement d'images et de mémorisation, le capteur d'images (8) se composant d'éléments semi-conducteurs (14) optoélectroniques disposés en forme de matrice sur un substrat (15) et d'un registre de lecture (12) qui capte les charges mémorisées dans les éléments semi-conducteurs (14) en fonction de l'intensité de rayonnement respectivement reçue, caractérisé en ce que
le registre de lecture (12) est agencé à l'intérieur de la surface du capteur d'images (8) de telle sorte que des éléments semi-conducteurs (14) se trouvent des deux côtés de l'axe longitudinal du registre de lecture (12), la surface de capteur est construite sous forme de rectangle à coins (11) arrondis ou biseautés et une forme arrondie est donnée aux endroits de l'étui (1) qui correspondent aux coins arrondis ou biseautés.
